# EUROPÄISCHE PATENTSCHRIFT

(11) **EP 4 295 679 B1**
(45) Veröffentlichungstag und Bekanntmachung des Hinweises auf die Patenterteilung: **13.08.2025**
(21) Anmeldenummer: 23180191.1
(22) Anmeldetag: 19.06.2023
(51) Int. Cl.: A01K 67/362, A01K 67/368

(54) **VORRICHTUNG UND VERFAHREN ZUR AUFZUCHT VON INSEKTENLARVEN**
DEVICE AND METHOD FOR REARING INSECT LARVAE
DISPOSITIF ET PROCÉDÉ D'ÉLEVAGE DE LARVES D'INSECTES

(30) Priorität: 20.06.2022 DE 102022115220
(43) Veröffentlichungstag der Anmeldung: 27.12.2023
(73) Patentinhaber: Fraunhofer-Gesellschaft zur Förderung der angewandten Forschung e.V., 80686 München (DE)
(72) Erfinder: KALUZA, Tim, 01189 Dresden (DE); CARSCH, Sebastian, 01277 Dresden (DE); EISENRAUCH, Vincent, 01127 Dresden (DE)
(74) Vertreter: Gottfried, Hans-Peter

(56) Entgegenhaltungen:
- EP-A1- 3 772 276
- CN-U- 214 282 828
- DE-T2- 69 312 740

## Beschreibung

Die Erfindung betrifft eine Vorrichtung und ein Verfahren zur Aufzucht von Insektenlarven.

Durch die aktuelle Form der Nutztierhaltung wird der steigende Proteinbedarf für die wachsende Weltbevölkerung nicht mit dem Erreichen nationaler wie internationaler Klimaziele vereinbar sein. Insbesondere die Reduktion des Fleischkonsums wird gefordert und als eine Lösungsstrategie für die Ernährung einer wachsenden Weltbevölkerung befürwortet. Daher ist die Erschließung alternativer Proteinquellen aktuell von hoher Bedeutung. Laut der Ernährungs- und Landwirtschaftsorganisation der Vereinten Nationen (FAO) bieten Insekten als alternative Proteinquelle eine gute Voraussetzung, um die Ernährung der wachsenden Bevölkerung zukünftig sicherzustellen und gleichzeitig deren Nachhaltigkeit zu erhöhen, da insbesondere stoffliche Ressourcen im Vergleich zur derzeitigen Fleischproduktion effizienter und nachhaltiger genutzt werden können. Insekten sind besonders aus Gründen der Nachhaltigkeit interessant, denn im Vergleich zu anderen tierischen Proteinen sind ihr CO₂-Fußabdruck sowie der Land- und Wasserverbrauch deutlich geringer.

In der Landwirtschaft gelten Insekten als wichtiger Faktor für die Entwicklung einer zirkulären Bioökonomie in der Lebens- und Futtermittelindustrie, da diese mit organischen Nebenströmen aus der Agrar- und Lebensmittelwirtschaft gefüttert werden können (z. B. Stroh, Treber und Trester) und die Exkremente und Futterreste (sogenannter Frass) aus der Insektenzucht bzw. der Aufzucht von Insektenlarven als hervorragend geeigneter Biodünger nutzbar sind.

Insektenzucht (englisch: *insect rearing* oder *insect farming*) ist die Vermehrung von Insekten außerhalb ihrer natürlichen Lebensumgebung zur Befriedigung menschlicher Bedürfnisse (Nutzinsekten), zum Beispiel als Lebensmittel (Speiseinsekten), als Futtermittel (Futterinsekten), zur Bestäubung von Kulturpflanzen oder zur biologischen Schädlingsbekämpfung. In der Regel werden Insekten dabei auf besonderen Nährsubstraten gehalten und vermehrt, eine eigentliche Zucht, also eine gezielte genetische Veränderung, ist damit im Normalfall nicht verbunden. Wenn nachfolgend von Zucht die Rede ist, geht es vorrangig um die Aufzucht von Insekten bzw. konkret von Insektenlarven.

Herausragende Bedeutung besitzt die Zucht von Speiseinsekten für die menschliche Ernährung vor allem in Südostasien und dort in Thailand, wo etwa 20.000 Farmer Heimchen (*Acheta domesticus,* eine Grillenart) und Sagowürmer (Larven des Rüsselkäfers *Rhynchophorus ferrugineus*) als Speiseinsekten für den menschlichen Verzehr züchten. Nach einer Aufstellung der Ernährungs- und Landwirtschaftsorganisation der Vereinten Nationen (FAO) werden in verschiedenen Teilen der Welt knapp 1700 Insektenarten zum menschlichen Verzehr genutzt, zumeist jedoch nicht gezüchtet, sondern aus ihren Wildvorkommen besammelt.

Als Speiseinsekten (ganz oder als Bestandteil in anderen Lebensmitteln) kommen die Larven des Mehlkäfers (*Tenebrio mollitor*), die europäische Wanderheuschrecke (*Locusta migratoria*)*,* Buffalowürmer (*Alphitobius diaperinus*)*,* verschiedene Grillenarten (*Acheta domesticus, Gryllodes sigillatus*), die männliche Larve der Honigbiene (*Apis mellifera male pupae*) und die Larven der schwarze Soldatenfliege (*Hermetia illucens*) in Betracht.

Die global stark wachsende industrielle Produktion von Insekten ist derzeit noch auf einem niedrigen Niveau, z. B. lag die Produktion von *H. illucens* zwischen 2014 und 2015 bei 7.000 - 8.000 Tonnen und 2016 bei 14.000 Tonnen. Mit der Zulassung der Larve des Mehlkäfers (*Tenebrio molitor*) 2021 als neuartiges Lebensmittel durch die europäische Behörde für Lebensmittelsicherheit (EFSA) ist das Insekt als Lebensmittel(zusatz) in der EU angekommen. In der EU wurden darüber hinaus bereits 2017 die ersten sieben Insektenarten (inklusive *T. molitor* und *H. illucens*) als Futtermittel in der Aquakultur zugelassen. 2021 erfolgte ebenfalls die Zulassung von Insektenproteinen in Haustierfutter sowie bei der Geflügel- und Schweinemast.

Die Druckschrift DE 693 12 740 T2 beschreibt ein Zuchtsystem für Insektenlarven, das aus einer Aufzuchteinheit mit getrennten Bereichen für Nahrung, Larven und Ausscheidungen besteht. Der Nahrungsbereich liefert eine geeignete Ernährung, während der darunterliegende Ausscheidungsraum Abfälle sammelt, um die Larven und Nahrung nicht zu stören. Senkrechte Trennwände oder Kantenstangen im Larvenraum ermöglichen eine optimale Verteilung und Anheftung der Larven, was eine Maximierung der Larvendichte erlaubt. Das System minimiert Material- und Arbeitsaufwand und ermöglicht eine präzise Feuchtigkeitskontrolle, wodurch es ideal für die wirtschaftliche Produktion von Viren (in den Insekten) oder anderen Produkten ist.

Die Druckschrift EP 3 772 276 A1 beschreibt eine Aufzuchtbox für Insektenlarven, die speziell für industrielle Zwecke optimiert wurde. Die Vorrichtung umfasst eine rechteckige Kammer mit Seitenwänden sowie abnehmbaren oberen und unteren Abdeckungen. In der Kammer befinden sich vertikale Stäbe, die den Larven als Kletter- und Haftflächen dienen und eine gleichmäßige Verteilung sowie optimale Fütterung sicherstellen. Eier werden auf der unteren Abdeckung platziert, während die obere eine Nahrungsquelle bietet; die Larven wandern von unten nach oben und entwickeln sich dort zu Puppen. Das System fördert eine homogene Entwicklung der Larven und ermöglicht eine effiziente Automatisierung, Lagerung und kostengünstige Produktion durch Einwegmaterialien.

Aus der Druckschrift CN 214282828 U ist eine Aufzuchtbox speziell für die Larven von Schmetterlingsinsekten bekannt. Die Box besteht aus einem Hauptbehälter mit einem herausnehmbaren Deckel und einem Filterboden, der die Larven von ihren Exkrementen trennt. Unter dem Filter befindet sich ein ebenfalls entfernbarer Auffangbehälter, der die Larvenexkremente sammelt, um eine Verunreinigung des Futters zu vermeiden. Die Larven können über ein speziell gestaltetes Netz, das als Futterplattform dient, Nahrung aufnehmen. Diese Konstruktion verhindert die Kontamination des Futters durch Exkremente und reduziert das Risiko von Schimmelbildung, was die Gesundheit der Larven fördert und gleichzeitig die Reinigung vereinfacht.

Speiseinsekten wie Wanderheuschrecken, Hausgrillen (Heimchen), Mehlwürmer oder Buffalowümer werden häufig in professionellen, spezialisierten Insektenzuchtbetrieben bzw. Insektenfarmen gezüchtet. Haltung, Aufzucht und Futter unterscheiden sich je nach Insektenart. Da Speiseinsekten zumeist in Farmen innerhalb von Gebäuden produziert werden, lassen sich die Faktoren für die Aufzucht genau kontrollieren. So können Temperatur, Feuchtigkeit, Helligkeit, Lautstärke und mehr genau überwacht und an die Bedürfnisse der Tiere angepasst werden. Viele Schritte in den Farmen lassen sich automatisieren. So kontrollieren Sensoren die Faktoren wie Temperatur oder Feuchtigkeit und optimieren diese automatisch.

Nach Erreichen des gewünschten Entwicklungsstadiums und der gewünschten Größe werden die Insekten meist durch Absenken der Temperatur schonend getötet. Anschließend werden sie meist gefriergetrocknet und entweder unzerkleinert als tiefgefrorene, frische Speiseinsekten verpackt zum Verkauf angeboten oder aber zu Insektenmehl bzw. Insektenpulver weiterverarbeitet. Dieses bildet die Grundlage für zahlreiche Produkte wie Insekten-Nudeln, Insekten-Brot oder Insekten-Bratlinge. Auf die Zucht von Speiseinsekten ist auch die vorliegende Erfindung vorrangig gerichtet, kommt jedoch auch für andere Nutzungsformen in Betracht.

Die Züchtung von Insekten ist auch als Tierfutter im privaten Bereich in der Aquaristik und Terraristik weit verbreitet, wo Heimchen, Mehlwürmer (Larven des Mehlkäfers *Tenebrio molitor*) und zahlreiche andere Arten als Futtertiere gezüchtet werden. Daneben ist die Insektenzucht für die Bestäubung von Nutzpflanzen und der Einsatz gezüchteter Insekten in der biologischen Schädlingsbekämpfung von Bedeutung. Für diese Nutzungsformen ist die vorliegende Erfindung nicht vorgesehen.

Die Produktion von Insekten erfolgt bislang als diskontinuierlicher Batch-Prozess in Mastkisten. Durch die geringen Schichtdicken können optimale Lebensbedingungen für Insektenlarven wie denen des Mehlkäfers *Tenebrio Molitor* garantiert werden. Die stapelbaren Kisten ermöglichen zudem eine hohe Flächeneffizienz und erschweren eine unkontrollierte Ausbreitung von Pathogenen im Produktionssystem. Nachteilig ist der hohe, teilweise rein manuelle Monitoringaufwand, das damit verbundene Kistenhandling und entsprechende manuelle Arbeit. Bekannte Optimierungen des Produktionssystems adressierten das automatisierte Kistenhandling und das sensorgestützte Monitoring. Vielversprechende optische Systeme können bei *T. Molitor* nicht problemlos eingesetzt werden, da das Insekt vom Futtersubstrat sowie von dessen Frass (Exuvie und Exkremente) bedeckt wird. Die Abdeckung behindert die kontinuierliche Überwachung der Insektenlarven. Die Biomassezunahme wird bei höherem Frass-Anteil reduziert. Aus diesem Grund wird in der konventionellen Mast der Insektenfrass abgesiebt und nachgefüttert. Eine Klasseneinteilung nach Entwicklungsstadium wäre hier möglich, erfolgt aber in der Regel nicht, da der Aufwand eines mehrphasigen Siebvorgangs derzeit zu hoch ist. Dies führt bei der Ernte zu einem inhomogeneren Ergebnis hinsichtlich des idealen Entwicklungstands der Insektenlarven. Darüber hinaus entfallen beim intelligenten Füttern die sonst erforderlichen zusätzlichen Aufwendungen durch die Siebung und die Nachfütterung.

Als Futtersubstrat der Mehlkäfer-Larven kommen Trockensubstrate zum Einsatz. Obst oder Gemüse wird als Feuchtigkeitsspender verwendet. Die Kombination zu einem Vollfuttermittel gelingt bislang nicht, da ein erhöhter Feuchtigkeitsanteil zur Ausbreitung von unerwünschten Schädlingen (beispielsweise Milbenbefall) im Futtersubstrat führt.

Vorrichtungen und Verfahren zur Insektenzucht, insbesondere zur Aufzucht von Insektenlarven, sind aus dem Stand der Technik bekannt. Nach der Druckschrift DE 10 2019 121 102 B3 erfolgt nach der Aufzucht eine abschließende Siebung, jedoch keine stetige, vom Entwicklungsgrad abhängige Trennung von Larven und Futtermittel. Es wird die Integration einer Futter-Dosierstation, von Analyse und Speicher in eine Anlage zur Insektenzucht beschrieben.

Die Druckschrift DE 20 2004 011 745 U1 offenbart ein Behältnis zur Beobachtung von Insekten, jedoch ohne das Ziel einer industriellen Zucht. Zur Trennung der Zuchtinsekten ist ein "Burggraben" vorgesehen.

Aus der Druckschrift DE 304 97 42 C2 ist die Aufzucht von Insekten für die Schädlingsbekämpfung bekannt, speziell auf die Aufzucht von Motten ausgelegt. Es ist jedoch keine gezielte Mast von Larven vorgesehen. Insbesondere wird die Produktion von Insekteneiern offenbart, jedoch keine Fütterung und keine gezielte Larvenernte.

Aus der Druckschrift DE 20 2018 107 092 U1 ist ein Zuchtschrank mit Schubladen bekannt, der als geschlossenes Regalsystem für die Aufzucht von Insekten in Kulturgefäßen dient. Er ermöglicht eine gezielte Konditionierung der Luft in dem Zuchtschrank. Ein Fluid wird als thermisches Transportmittel genutzt.

Die Druckschrift DE 10 2020 004 957 A1 beschreibt ein Verfahren und eine Vorrichtung zur Larvenentwicklung von Insekten und deren Trennung vom Substrat, speziell für *Hermetia Illucnes* (Schwarze Soldatenfliege). Die Trennung erfolgt in einem Nass-Siebverfahren, z. B. in einer Spannwell-Siebmaschine. Ein erhöhter Druck im Substrat, hervorgerufen durch die Siebung, könnte die Larven schädigen. Die Siebung ist zeitlich nach der Aufzucht geplant, eine kontinuierliche Zucht und Trennung ist nicht vorgesehen. Auch eine wiederholte Siebung für ein besseres Binning (Klassierung, Separierung) ist nicht geplant, eine stetige Trennung von Larven und Futtermittel ist nicht möglich. Weiterhin ist keine kontinuierliche bzw. gezielte oder adaptive Fütterung vorgesehen.

Auch die Druckschriften US 1 108 277 A, DE 164629 A und US 2018/0084763 A1 befassen sich mit der Fütterung von Insekten, jedoch speziell von Bienen. Diese werden mit Sirup niedriger Viskosität gefüttert. Bei anderen Insekten bzw. deren Larven, insbesondere beim Mehlwurm, ist ein Sirup oder auch ein Gelee jedoch zu dünnflüssig und die Larven könnten bereits durch die Benetzung der Oberfläche mit Feuchtigkeit ertrinken. Zu hohe Feuchtigkeit kann auch zu Schimmelbildung führen, was für die Insektenzucht fatal ist.

Die in den Druckschriften US 1 108 277 A, DE 164629 A und US 2018/0084763 A1 beschriebenen Einrichtungen zur Bienenfütterung gehen von einer Art Trogfütterung aus. Der Futterplatz wird von den Bienen nur zur Nahrungsaufnahme aufgesucht. Ein Ort, auf dem die Insekten den gesamten relevanten Lebenszyklus verbringen und dabei Futter aufnehmen können, wird nicht vorgeschlagen. Die bei der Larvenaufzucht erforderliche Entsorgung der Nebenströme wie Kot, Frass und Häute wird in den Druckschriften nicht behandelt.

Das in den Druckschriften US 1 108 277 A, DE 164629 A und US 2018/0084763 A1 offenbarte technische Prinzip funktioniert nicht beim Einsatz von höher- und hochviskosen oder gar pulverförmigen Stoffen, wie sie für die Fütterung von Insektenlarven verwendet werden. Diese Futterstoffe strömen nicht durch den Docht oder die anderen Vorrichtungen, die in den Druckschriften genannt sind. Für insbesondere nachhaltige Futtermittel für die Larvenaufzucht mit dem Ziel der Proteinproduktion werden organische "Restströme" verwendet, wie z. B. Bäckereiabfälle (Brot, Kuchen, Gebäck), Gemüse- und Obstabfälle, Eier- und Milchprodukte (Milch, Käse, Joghurt), Getreideabfälle aus Brauereien, Biertreber, Kaffeepulpe, Rübenpulpe, Pülpe aus Zuckerfabriken, Kartoffelpülpe und Kartoffelchips aus der kartoffelverarbeitenden Industrie, Kartoffelpülpe aus der Kartoffelmehlindustrie, Maiskleber aus der Maisstärkeindustrie. Typisch sind auch mehl- und getreidehaltige Futtermittel. Alle diese Stoffe können, auch nach Aufbereitung, nicht über die im Stand der Technik vorgeschlagenen Vorrichtungen dosiert werden.

Neben dem auf einer Kapillarwirkung basierenden Docht dosiert zudem bei der in der Druckschrift US 1 108 277 A beschriebenen Vorrichtung ein Schieber das Futter für die Bienen. Für eine Aufzucht von Insektenlarven ist ein solches System nachteilig, da immer möglichst viel Futter zur Verfügung stehen sollte, damit die Larven jederzeit gewünscht, denn die Insektenlarven leben auf der Fütterungseinrichtung bis zur Schlachtung.

Der Stand der Technik bei der Aufzucht von Insektenlarven sieht regelmäßig einen Chargenbetrieb vor, bei dem ein Binning, ein Sortieren nach gleicher Güte und Art, angestrebt wird. Bei Insekten ist es das Ziel, möglichst Insektenlarven mit ähnlichem Entwicklungsstand und Potential zu erhalten bzw. zu sortieren. Allerdings ist unter dieser Prämisse keine Möglichkeit zur kontinuierlichen Zucht insbesondere unter den vorgenannten Anforderungen bekannt. Auch aufgrund der Neuartigkeit von Insekten im Lebens- und Futtermittelbereich und dem Mangel an spezifischen Automatisierungsprozessen ist die Produktion kostenintensiv, vor allem durch einen hohen Anteil manueller Arbeit und Eingriffe.

Es ist daher Aufgabe der vorliegenden Erfindung, eine Vorrichtung zur Aufzucht und Ernte von Insektenlarven anzubieten, bei der eine dosierte Fütterung ermöglicht wird und eine schonende Trennung insbesondere von Futter und Insektenlarven erfolgen kann.

Die Aufgabe wird gelöst durch eine Vorrichtung zur Aufzucht von Insektenlarven gemäß Anspruch 1, bei der ein Futterraum von einem Aufzuchtraum durch eine Barriere getrennt ist. Die Barriere weist eine Aufzuchtfläche, auf der die Insektenlarven leben, und eine der Aufzuchtfläche gegenüberliegende Futterraumfläche auf, an der ein beispielsweise höherviskoses oder gelartiges Futtermittel für die Insektenlarven anliegt. Die Barriere zudem weist eine Vielzahl von Öffnungen auf, die durch ihre Größe eine Passage der Insektenlarven durch die Barriere verhindern, jedoch den Zugang der Insektenlarven zum Futtermittel gewährleisten. Insbesondere ist dazu das Futtermittel so beschaffen, dass es so weit in die Öffnungen eindringt, dass es für die Insektenlarven erreichbar wird, ohne dass diese jedoch ganz in das Futter hineingelangen können.

Die Barriere mit den Öffnungen ist bevorzugt als ein Lochblech, ein Gittergeflecht oder ein Abstandsgewirke ausgeführt. Das Abstandsgewirke kann als eine 2D- oder 3D-Wirkstruktur, wie sie aus der Textiltechnik bekannt ist, ausgeführt sein. Der maximale Durchmesser der Löcher der Barriere sollte daher 1,5 mm bis 2 mm nicht überschreiten, da eine typische Junglarve einen Durchmesser von 3 mm aufweist. Der Durchmesser kann aber auch etwas größer sein, um Futter höherer Viskosität leichter passieren zu lassen. Wenn das Futter kontinuierlich gefördert wird, können sich die Insektenlarven nicht durchfressen, sodass auch ein größerer Öffnungsdurchmesser als der Insektendurchmesser gewählt werden kann.

Das Abstandsgewirke bewirkt auch, dass das Futtermittel bzw. der Futterbrei in einer konstanten Position in der Barriere für die Insektenlarven zugänglich gehalten wird, ohne dass permanent nachgepumpt werden muss. Hierzu tragen beispielsweise ein Durchmesser und eine Länge der Öffnungen bei, die abgestimmt sind auf die Viskosität des Futterbreis.

Vorzugsweise ist die Barriere gegenüber der Horizontalen geneigt, insbesondere im Winkel von 5 bis 30°, wobei der Winkel vom Material der Oberfläche und vom Einsatz von Vibrationen abhängt. Auf normal rauen metallischen Materialen (nicht spiegelglatt und nicht extra aufgeraut) ist ein Winkel mit 10° bei Vibration zu empfehlen, ohne Vibration sollten 25° gewählt werden. Bei glatten Kunststoffen reichen bereits die 5° mit Vibration aus. Bei faserbasierten Materialen, wie z. B. Kartonage oder Textil, sind vertikale niederfrequente, stoßartig harte Impulse geringer Amplitude notwendig. Hierbei lösen sich die Maden, die Insektenlarven, dann ab einer Neigung von 30° ab, da sie danach schnell genug fallen und sich nicht sofort wieder festkrallen können. Ohne Vibration lösen sich manche Maden auch nicht bei einer Drehung um 180° ab. Die nachfolgende Tabelle zeigt die bevorzugten Neigungen unter verschiedenen Bedingungen.

**Tabelle 1: Neigung der Barriere unter verschiedenen Bedingungen**

| Oberfläche der Barriere | Einsatz von Vibration | ohne Vibration |
|---|---|---|
| Gebürsteter Edelstahl | 10° | 25° |
| Feine Kartonage | 30° | Festhalten der Maden an Fasern |
| Textil | 35° | Festhalten der Maden an Fasern |
| PP glatt | 5° | 20° |
| Verzinktes Blech | 10° | 25° |

Die Neigung erleichtert oder ermöglicht eine Trennung von Abscheidungen, Frass und Häuten von der Oberfläche, die die Aufzuchtfläche bildet. Die Trennung erfolgt z. B. durch Absaugen, insbesondere mittels einer Absaugeinrichtung, oder Abschütteln, ggf. durch den Einsatz von Vibration. Frass ist eine gebräuchliche Beschreibung für auch als Dünger nutzbare Exkremente der Insekten. Der Frass ist relativ trocken, fast staubförmig und sehr fein. Alte Chitinhäute sind auch Exkremente, zählen aber nicht zum Frass.

Ab einem Neigungswinkel von circa 10° werden die Reststoffe wie Häute durch die Schwerkraft und die Eigenbewegung der Insektenlarven, die zufällig gegen die Reststoffe stoßen, abtransportiert, indem die Haftreibungskräfte gegenüber dem Untergrund überwunden werden. Es wird eine Roll- oder Gleitbewegung der Reststoffe hervorgerufen.

Nach einer vorteilhaften Ausführungsform ist die Barriere so ausgestaltet bzw. mit einem entsprechenden Antrieb versehen, dass sie zur Ernte der Insektenlarven in Vibration versetzt werden kann. Eine individuelle Ernte der Insektenlarven wird auf diese Weise einfach möglich, da sich überraschend gezeigt hat, dass sich die Insektenlarven vor der Verpuppung nicht mehr an der Barriere festbeißen und sich daher leicht abschütteln und dadurch separieren lassen. Um die Larven der Insekten, im Weiteren kurz Insektenlarven genannt, geht es bei der Insektenzucht im Sinne der vorliegenden Erfindung vorrangig, da für die Insektenlarven in diesem Entwicklungsstadium und insbesondere vor der Verpuppung die beste Nutzbarkeit vorliegt.

Vor der Verpuppung haben die Insektenlarven ihre maximale Masse erreicht und damit liegt ein optimaler Erntezeitpunkt vor. In diesem Zustand lässt die Aktivität der Insektenlarven stark nach, sie halten sich kaum noch an der Aufzuchtfläche fest und können leicht geerntet werden. Die Haltekraft schwankt je nach Material und Zeitpunkt.

Eine sporadische Vibration kann auch genutzt werden, um ein "Verstopfen" der Aufzuchtfläche zu verhindern. Zu einem derartigen "Verstopfen" kommt es, wenn Insektenlarven dauerhaft an einer Öffnung festgebissen bleiben und andere Insektenlarven, die keine Öffnung besetzen konnten, nur unzureichend an das Futtermittel gelangen.

Es hat sich als vorteilhaft erwiesen, wenn die Barriere eine Beschichtung aufweist oder aus einem Material besteht, das Schimmel und Abrieb verhindert. Dabei ist darauf zu achten, dass die Beschichtung und das Material keine nachteiligen Effekte auf die Insektenlarven hervorrufen. Derartige Materialien sind aus dem Stand der Technik bekannt und könnten vom Fachmann entsprechend ausgewählt werden.

Vorteilhafterweise ist eine Pumpe zur Zufuhr des Futtermittels in den Futterraum vorgesehen. Diese sorgt für den Nachschub des Futtermittels bzw. den ausreichenden Druck im Futterraum, sodass an allen Öffnungen stets Futtermittel anliegt und eine optimale Versorgung gewährleistet ist. Ein Futtermittel bzw. Futterbrei mit rheologischen Eigenschaften, die eine Pumpfähigkeit sicherstellen, bzw. eine angepasste Pumpentechnik, beispielsweise eine Exzenterschneckenpumpe, sind die Voraussetzung für eine Automatisierung der Aufzucht von Insektenlarven. Der Einsatz ist daher nur beschränkt möglich.

Nach einer vorteilhaften Weiterbildung ist die Barriere beweglich ausgeführt, beispielsweise als Band, führt kontinuierlich Frass und Häute ab und wirft Insektenlarven zur Ernte aus. Eine sich in dieser Weise stetig weiterbewegende, flexible Barriere ist vorteilhafterweise als ein endloses Abstandsgewirke, wie oben beschrieben, ausgeführt. Wenn sich die derart ausgebildete Barriere langsam bewegt, besteht die Möglichkeit, dass die Insektenlarven sich immer in Richtung des Futtermittels, entgegen der Laufrichtung der Barriere, bewegen und deshalb an der Aufzuchtfläche bleiben. Dies betrifft Insekten, die noch an der Nahrungsaufnahme interessiert sind. Sobald sie diese einstellen und nicht mehr zum Futter zurückkehren, fallen sie am Ende des Bands ab, wodurch sich ebenfalls eine Separation erntereifer Insektenlarven erreichen lässt.

Eine Alternative zu der Barriere, die mit dem Aufzuchtbehälter fest verbunden ist, stellt eine Barriere dar, die als ein unterer Abschluss einer weiterhin zumindest seitlich geschlossenen Insektenbox ausgebildet ist. Diese Barriere sitzt mit der Futterraumfläche auf dem Futtermittel auf, wodurch dieses durch die Öffnungen hindurch von der Aufzuchtfläche her durch die Insektenlarven erreichbar ist. Damit liegt die Insektenbox auf dem Futter auf, kann aber dort, wegen der hohen Viskosität des Futtermittels oder weil es sich ohnehin um ein Pulver oder Granulat (im Folgenden stets als Pulver bezeichnet) handelt, nicht versinken.

Für den Weitertransport der Insektenbox für die kontinuierliche Erreichbarkeit des Futtermittels sinkt die Insektenbox mit dem Verbrauch des Futtermittels in diesem ab. Alternativ hierzu ist eine Vibrationseinrichtung mit der Insektenbox und/oder dem Aufzuchtbehälter verbunden und unterstützt das Absinken der Insektenbox im Futtermittel durch Vibrationen.

Die Nahrung für die Insektenlarven ist meist kein typisches Fluid, sondern ein höherviskoses Fluid oder ein pulverförmiges, heterogenes Stoffgemisch, ein granulierter Feststoff, der einen Feuchtigkeitsanteil besitzt. Als Beispiel kommen trockene gemahlene Brötchen mit etwas Wasser gemischt in Betracht. Solche Futtermittel sind besonders vorteilhaft durch die Insektenbox verfütterbar.

Nicht Teil der vorliegenden Erfindung ist ein Futtermittel für Insekten, das die benötigten Nährstoffe und Wasser enthält. Das Futtermittel ist nach einer ersten Ausführungsform zu einem höherviskosen oder gelartigen Fluid aufbereitet, das durch seine Oberflächenspannung von der Futterraumfläche her in die Öffnungen eindringt, aber nicht an der gegenüberliegenden Aufzuchtfläche herausläuft. Es bleibt stattdessen in den Öffnungen für die Insektenlarven zur Futteraufnahme erreichbar.

Alternativ ist das Futtermittel zu einem feuchten Pulver mit einem Wassergehalt zwischen 60 % und 90 %, vorzugsweise und im Interesse optimaler Bedingungen für die Insektenlarven einem Wassergehalt zwischen 70 % und 80 % aufbereitet. Bei diesem Feuchtegehalt lebt die Larve der Schwarzen Soldatenfliege in ihrem "Wohlfühlbereich".

Die Viskosität des höherviskosen oder gelartigen Fluids beträgt zwischen 10⁴ MPa*s und 10⁸ MPa*s. Es sind auch pulverförmige Futtermittel vorgesehen, die eine Partikelgröße aufweisen, die unter der lichten Weite der Öffnungen in der Barriere liegt, damit sie von den Insektenlarven erreicht werden können, wenn sie in die Öffnungen eindringen. Das pulverförmige Futtermittel kann aus trockenen oder feuchten Partikeln, insbesondere auch aus einem inhomogenen Materialgemisch bestehen. Abhängig ist dies nicht zuletzt von der verfügbaren Reststofffraktion, zumeist unterschiedliche Abfälle aus der Lebensmittelherstellung, die als Futtermittel aufbereitet und zur Aufzucht der Insektenlarven verwendet wird.

Das Futtermittel enthält alle benötigten Nähstoffe und das Wasser für die Insekten. Die Zusammensetzung kann nach dem Entwicklungstand der Insektenlarven und auch zur Beeinflussung des Nährstoffgehalts im Produkt angepasst werden. Eine weitere Lösung betrifft auf dieser Basis die Verwendung eines höherviskosen, gelartigen oder pulverförmigen Futtermittels für Insekten, das die benötigten Nährstoffe und Wasser, auch ohne gesonderte Luftkonditionierung, enthält, in einer Vorrichtung, wie sie zuvor beschrieben wurde. Bei der Luftkonditionierung, insbesondere auch eines Gebäudes, werden alle technischen Maßnahmen ergriffen, die die Raumluft so behandeln, dass die Luft im optimalen Zustand für die Gesundheit der Bewohner des Gebäudes ist. Die wichtigsten Parameter für eine gesunde Raumluft sind die Temperatur, der Feuchtegehalt und der Feinstaubanteil der Luft. Daraus ergeben sich jeweils die technischen Bereiche der thermischen Klimatisierung, der Be- sowie Entfeuchtung und der Luftreinigung. Dies trifft entsprechend auch auf die erfindungsgemäße Vorrichtung zu.

Das Futtermittel ist vorzugsweise zu einem höherviskosen oder gelartigen Fluid aufbereitet, das durch seine Oberflächenspannung von der Futterraumfläche her in die Öffnungen eindringt, aber nicht an der gegenüberliegenden Aufzuchtfläche herausläuft. Es bleibt stattdessen in den Öffnungen für die Insektenlarven zur Futteraufnahme erreichbar. Alternativ handelt es sich um ein pulverförmiges Futtermittel, basierend auf einem trockenen oder insbesondere feuchten Material. Dieses weist einen Wassergehalt von 60 % bis 90 %, der zugleich den technisch realisierbaren Bereich darstellt. Demgegenüber liegt der bevorzugte Bereich, vor allem bei der Aufzucht der Schwarzen Soldatenfliege, zwischen 70 und 80 %.

Die Aufgabe der Erfindung wird ebenfalls gelöst durch ein Verfahren zur Aufzucht von Insektenlarven gemäß Anspruch 10, bei dem das oben beschriebene Futtermittel einer Vorrichtung zur Aufzucht von Insektenlarven kontinuierlich in der Weise in deren Futterraum zugeführt wird, dass es durch die Öffnungen in der Barriere stets für die Insektenlarven verfügbar ist. Dabei werden kontinuierlich Frass und Häute abgeführt sowie Insektenlarven vor der Verpuppung geerntet, da diese sich nicht festbeißen und sich abschütteln lassen. Dies kann unterstützt werden durch die Ausbildung der Oberfläche der Aufzuchtfläche, deren Neigung, durch eine gezielte und erzwungene Luftströmung entweder mit Über- oder Unterdruck sowie weiterhin durch leichte Vibration oder temporäres Kippen des Behältnisses.

Die diskontinuierliche Zufuhr von Futtermittel bzw. Abfuhr von Frass und Häuten erfolgt insbesondere bei Einsatz der Insektenbox. Die Entfernung von Frass und Häuten kann hier beispielsweise durch Absaugen mittels der Absaugeirichtung erfolgen. Die Futterzufuhr erfolgt aus Sicht der Insektenlarven kontinuierlich, während die Insektenbox weiter in den Futterraum einsinkt, wenn sich die Schicht an Futtermittel verringert. Die Zufuhr des Futtermittels erfolgt aus Sicht des Bedieners diskontinuierlich, wenn das Futtermittel im Futterraum nachgefüllt wird.

Durch eine definierte Neigung der Barriere im Winkel von 5° bis 30° je nach den Randbedingungen, wie zuvor in Detail erläutert, gegenüber der Horizontalen werden Insektenlarven, die sich vor der Verpuppung kaum noch an der Aufzuchtfläche festhalten, abtransportiert. Die Resthaftung wird aufgehoben durch:
- Vibration: Die Vibration ist insbesondere charakterisiert durch induzierte niederfrequente Schwingung in horizontaler und oder vertikaler Richtung, gleichmäßig oder wiederkehrend (ähnlich konventioneller Siebanlagen).
- Impulse: Gezielte einmalige oder wiederkehrende Bewegung der Barriere bringt die die Insektenlarve zum Abrollen oder Abfallen; eine Absaugung erfasst die Insektenlarven, die sich an der Oberfläche befinden, unbeweglich verharren und sich nicht an der Oberfläche festhalten.
- Kippen: Das Aufzuchtbehältnis kann gekippt oder kurzzeitig gedreht werden, da währenddessen das Futter durch die Barriere fixiert wird. Aktive, hungrige Insektenlarven sind im Futter und damit an der Barriere durch das Fressen "verankert", ausgewachsene Insektenlarven fressen nicht mehr und fallen ab.

Die Neigung ist insbesondere bei der fest mit dem Aufzuchtbehältnis verbundenen Barriere vorgesehen, nicht hingegen beim Einsatz der Insektenbox. Diese stellt ihre Position beim Absinken mit dem Futtermittel stochastisch ein.

Die Vorteile der vorliegenden Erfindung bestehen unter anderem darin, dass die Insektenlarven einfacher beobachtet und untersucht werden können, da sie nun ständig von der Oberfläche, der Aufzuchtfläche her sichtbar sind. Zudem können abgestoßene Insektenhäute leichter separiert (z. B. abgesaugt) werden, da sie vom Futter getrennt sind. Der Frass der Insektenlarven ist ebenfalls leichter zu separieren, da er nicht mit dem Futter vermengt ist. Es ist von einem schnelleren Wachstum der Insektenlarven auszugehen, weil das Futter leichter von ihnen aufgenommen werden kann. Die Ernte der Insektenlarven kann individuell erfolgen, da die Insektenlarven sich vor der Verpuppung von der Barriere ablösen und leicht entnommen werden können. Die Erfindung schafft die Voraussetzungen, um den Prozess der industriellen Insektenzucht zu automatisieren, zumindest aber um den Prozess kontinuierlich zu gestalten und von der herkömmlichen diskontinuierlichen Arbeitsweise abzugehen.

Mit der vorliegenden Erfindung wird eine Technologie angeboten, mit der die effektive, effiziente und sichere intelligente Fütterung von Insektenlarven realisiert und die artgerechte und ökonomische effiziente Haltung ermöglicht wird. Das erfindungsgemäße Konzept basiert auf einer Trennung zwischen Futter und Insekten.

Durch das intelligente Füttern kann ein Vollfuttermittel verwendet werden. Da die Insektenlarven beim intelligenten Füttern weder dauerhaft in Kontakt mit dem Futtersubtrat oder dem Insektenfrass stehen, kann das Risiko der Ausbreitung von Pathogenen erheblich reduziert werden.

Die vorliegende Erfindung liefert durch ihren Ansatz zur bedarfsgerechten Fütterung von Insektenlarven die Grundlage für einen effizienten Aufzuchtprozess. Sie ermöglicht den Paradigmenwechsel von der bislang diskontinuierlichen, chargenweisen Produktion hin zu einem kontinuierlichen, adaptiven Aufzuchtprozess. Besonderes Augenmerk liegt dabei auf der kontinuierlichen Abführung und dem Durchsatz einer bedarfsgerechten Menge des Futtermittels als ein Vollfutters an den Insektenlarven vorbei auf eine Weise, bei der die Insektenlarven auf der Fütterungsebene, der Aufzuchtfläche, verbleiben und für analytische Methoden detektierbar bleiben. Das Vollfutter muss dabei sowohl die ernährungsphysiologischen Anforderungen der Insektenlarven erfüllen als auch die verfahrenstechnische Umsetzung der intelligenten Fütterung ermöglichen.

Beim Einsatz der Insektenbox erfolgt die kontinuierliche Verfahrensführung quasikontinuierlich, indem eine Reihe von Insektenboxen gebildet wird. In die erste Insektenbox werden die Junglarven eingesetzt. Danach wird sie weiterbewegt und eine neue erste Box kommt zum Einsatz. Währenddessen wachsen die Insektenlarven heran bis zur Erntereife, wozu sie aus der Insektenbox, wenn sie an die letzte Position in der Reihe gelangt ist, entnommen werden.

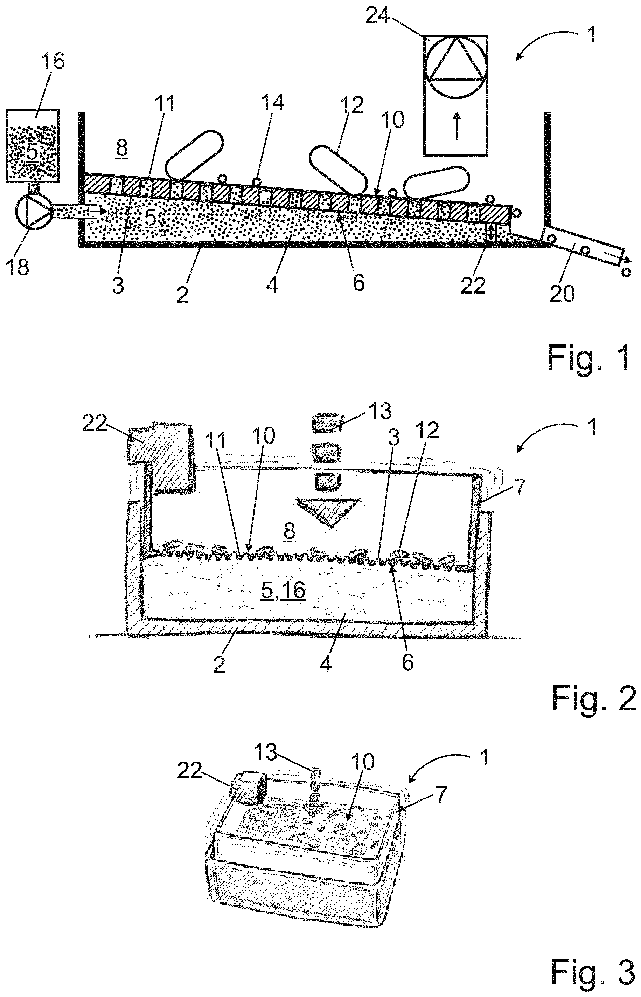

Anhand der Beschreibung eines Ausführungsbeispiels und seiner Darstellung in der zugehörigen Zeichnung wird die Erfindung nachfolgend näher erläutert. Fig. 1 zeigt schematisch eine perspektivische Ansicht einer Ausführungsform einer erfindungsgemäßen Vorrichtung 1 zur Aufzucht von Insektenlarven, mit einem Aufzuchtbehältnis 2, in dem eine Barriere 3 angeordnet ist. Die Barriere 3 trennt einen Futterraum 4 von einem Aufzuchtraum 8. In den Futterraum 4 weist eine Futterraumfläche 6, eine erste Oberfläche der Barriere 3, und in den Aufzuchtraum 8 weist eine Aufzuchtfläche 10, eine zweite Oberfläche der Barriere 3, auf der die Insektenlarven 12 leben.

Der Futterraum 4 ist angefüllt mit einem Futtermittel 5, in der bevorzugten Ausführungsform ein mittels einer Pumpe 18 pumpbares Fluid, das vorzugsweise hochviskos oder gelartig ist. Dadurch kann es in Öffnungen 11 der Barriere 3 vom Futterraum 4 her eindringen, läuft jedoch nicht aus den Öffnungen 11 in den Aufzuchtraum 8 hinein. Dies wird erreicht aufgrund einer Abstimmung von Parametern wie der Größe der Öffnungen 11, dem Druck im Futterraum 4 und der rheologischen Beschaffenheit des Futtermittels 5. Zusätzlich zu den vorgenannten Bedingungen kann auch ein Kapillareffekt in den Öffnungen 11 zum Tragen kommen, um eine konstante Zufuhr von Futtermittel 5 in den Öffnungen 11 und damit eine kontinuierliche Ernährung der Insektenlarven 12 gewährleisten. Das Futtermittel 5 wird in einem Futtermitteltank 16 bevorratet und kann bedarfsgerecht durch die Pumpe 18 von dort entnommen und in den Futterraum 4 hinein gefördert werden, soweit es pumpfähig ist.

Auf der Aufzuchtfläche 10 leben die Insektenlarven 12, die Insekten in ihrem Larvenstadium, bei der bevorzugten Verwendung der erfindungsgemäßen Vorrichtung 1. Sie ernähren sich über die Öffnungen 11, an denen bzw. an der Aufzuchtfläche 10 sie sich festhalten, um den Zugang zum Futtermittel 5 zu sichern. Verschiedene Abscheidungen, insbesondere Frass 14, rollt von der geneigten Aufzuchtfläche 10 ab, was durch eine Vibrationseinrichtung 22 unterstützt werden kann, und gelangt in einen Abwurf 20. Die staubartigen Exkremente können beispielsweise als Dünger Verwendung finden.

Haben die Insektenlarven 12 die gewünschte Größe erreicht, lässt die Intensität des Festhaltens an der Aufzuchtfläche 10 nach und sie können leicht geerntet werden. Dies erfolgt bei der dargestellten Ausführungsform durch eine Absaugeinrichtung 24, wobei die Insektenlarven 12 durch einen Luftstrahlstrom von der Aufzuchtfläche 10 mittels einer Absaugeinrichtung 24 abgesaugt werden. Auch dieser Vorgang kann durch die Vibrationseinrichtung 22 unterstützt werden, um im Moment des Absaugens etwaige Verankerung der Insektenlarven 12 auf der Aufzuchtfläche 10 kurzzeitig aufzuheben oder zu verringern.

Neben der in Fig. 1 dargestellten Ausführung der Barriere 3 als im Aufzuchtbehältnis 2 fixiertes Element, beispielsweise ausgeführt als Lochblech, kommt alternativ eine bandförmige Ausführung in Betracht. Dabei bewegt sich die bahnförmige Barriere 3 langsam vorwärts und vom Futter weg, sodass die an der Futteraufnahme interessierten Insektenlarven 12 sich entgegen der Bewegungsrichtung wieder zum Futtermittel 5 hin bewegen. Durch die Bewegung der Barriere 3 wird der Frass 14 kontinuierlich abgeworfen und zugleich werden all jene Insektenlarven 12, die die Futteraufnahme beendet haben und sich auf ihr Puppenstadium vorbereiten, abgeführt. Eine Automatisierung der Insektenzucht ist jedoch mit beiden Ausführungsformen möglich.

Eine alternative Ausführungsform ist in den Figuren 2 und 3 dargestellt, wobei alternativ zur innerhalb des Aufzuchtbehälters 2 statisch positionierten, inaktiven Barriere 3, zu deren Futterraumfläche 6 das Futtermittel 5 aktiv zugeführt wird (vgl. Fig. 1) diese nun beweglich ausgeführt ist. Fig. 2 zeigt eine Ausführungsform mit beweglicher Barriere 3 in einer geschnittenen, Fig. 3 in einer perspektivischen schematischen Darstellung.

Während ein flüssiges oder pastöses Futtermittel 5, wie es beispielsweise bei der Aufzucht der Schwarzen Soldatenfliege eingesetzt wird, problemlos einer statisch positionierten Barriere 3 zugeführt werden kann, benötigt der *Tenebrio molitor* dagegen eher trockene Nahrung. Hierzu gehören zum Beispiel zerkleinertes altes Brot oder Haferflocken. Bei einem derartigen, trockenen Futtermittel 5 kann es zu größeren Problemen beim Transport durch die Pumpe 18 kommen.

Die Barriere 3 ist deshalb als eine aktiv und/oder passiv bewegliche Insektenbox 7 ausgeführt, an deren Unterseite die Barriere 3 angeordnet ist. Beispielsweise wird die Insektenbox 7 in einen Eurobehälter (auch Eurobox genannt) typischerweise mit dem Außenmaß 300 x 200 x 120 mm (Länge x Breite x Höhe) eingesetzt. Der Eurobehälter dient als Aufzuchtbehälter 2, wobei die Insektenbox 7 nur wenig kleiner ist, mit der Aufzuchtbehälter 2 die Insektenbox 7 im Inneren aufnehmen kann. Alternativ kann die Insektenbox 7 oder mehrere Insektenboxen 7 auch in einen größeren Futtervorrat eingesetzt werden, ungeachtet der Grundflächen beider Objekte.

Diesem Futtervorrat kann diskontinuierlich oder auch kontinuierlich weiteres Futter zugefügt werden. Damit wäre das bedarfsgerechte Füttern bei verringertem Arbeitsaufwand und ohne aufwändige und störanfällige Technik möglich. Darüber hinaus kann das Futtermittel 5 dem Entwicklungsstand der Insektenlarven 12 entsprechend verwendet werden, sodass Insektenboxen 7 mit jungen Insektenlarven 12 in einer Futterbox 4, einem großen Futterraum 4, oder dem Aufzuchtbehältnis mit entsprechendem Futter für junge Insektenlarven 12 sitzen und dann nach entsprechendem Wachstum diese Insektenboxen 7 in eine andere Futterbox 4 oder Aufzuchtbehältnis 2 mit Futtermittel 5 für ältere Insektenlarven 12 kommen.

Die Insektenboxen 7 liegt mit der Futterraumfläche 6 der Barriere 3 auf dem Futtermittel 5 auf und ist zunächst zumindest durch einen Teil der Öffnungen 11 für die Insektenlarven 12 zugänglich. Für die weitere Zugänglichkeit des Futtermittels 5 für auf der Aufzuchtfläche 10 lebende Insektenlarven 12 sind folgende Möglichkeiten vorgesehen:
- Möglichkeit A: Die stetige Versorgung der Insektenlarven 12 mit Futtermittel 5 erfolgt durch Schwerkraft und/oder eine Vibrationseinrichtung 22. Die Vibrationseinrichtung 22 als ein aktiv angesteuertes Element ist dazu direkt mit der Insektenbox 7 verbunden und versetzt diese in tiefer- oder höherfrequente Schwingung und führt zur Bewegung. Durch beide Effekte liegt das Futtermittel an den Öffnungen 11 an oder dringt in diese ein Stück ein.
- Möglichkeit B: Die Barriere 3 der Insektenbox 7 ist so gestaltet, dass die Insektenlarven 12 durch ihre eigene Nahrungsaufnahme die Bewegung der Insektenbox 7 nach unten, in das Futtermittel 5 hinein hervorrufen, weil sie das das Futtermittel 5 unter der Insektenbox 7 verzehren und damit einen Hohlraum schaffen, in den die Insektenbox 7 nach und nach hineingleitet und dadurch weiteres Futtermittel 5 für die Insektenlarven 12 erreichbar wird.
- Möglichkeit C: Eine Kombination der Möglichkeiten A und B, wobei durch eine Vibration nur einzelne Erhebungen auf der Oberfläche des Futtermittels 5 durch die Vibrationen ausgeglichen werden. In dem Fall ist nur kurzzeitig eine von außen induzierte Bewegung mittels der Vibrationseinrichtung 22 notwendig und im Übrigen sinkt die Insektenbox 7 weiter ab durch den Verzehr des Futtermittels 7 durch die Insektenlarven 12.
- Möglichkeit D: Die Futterbox bzw. das Aufzuchtbehältnis 2 wird aktiv in Bewegung gesetzt, beispielsweise durch eine Vibrationseinrichtung 22 und die Insektenboxen 7 verharren bzw. bewegen sich durch den Verzehr des Futtermittels 5.

Vorteilhaft ist ein flexibler Einsatz des Futtermittels 5, da sich die zuvor beschriebenen Möglichkeiten nicht nur bei trockenem, pulverförmigem Futtermittel 5, sondern ebenfalls gut bei feuchterem, pastösem Futtermittel 5 anwenden lassen. Dies ermöglicht eine deutlich einfachere Vorrichtung, die flexible Verwendung unterschiedlicher Futtermittel und löst das Problem modular auf.

### Bezugszeichenliste

- 1: Vorrichtung
- 2: Aufzuchtbehältnis
- 3: Barriere
- 4: Futterraum, Futterbox
- 5: Futtermittel
- 6: Futterraumfläche
- 7: Insektenbox
- 8: Aufzuchtraum
- 10: Aufzuchtfläche
- 11: Öffnungen
- 12: Insektenlarve
- 13: Absenkrichtung
- 14: Frass
- 16: Futtermitteltank
- 18: Pumpe
- 20: Abwurf
- 22: Vibrationseinrichtung
- 24: Absaugeinrichtung

## Patentansprüche

1. Vorrichtung (1) zur Aufzucht von Insektenlarven, umfassend ein Aufzuchtbehältnis (2) und darin angeordnet eine Barriere (3), einen Futterraum (4) und einen Aufzuchtraum (8), wobei der Futterraum (4) von dem Aufzuchtraum (8) durch die Barriere (3) getrennt ist, wobei die Barriere (3) eine Aufzuchtfläche (10) und eine der Aufzuchtfläche (10) gegenüberliegende Futterraumfläche (6) aufweist, an der ein mit einer Viskosität zwischen 10⁴ MPa*s und 10⁸ MPa*s höherviskoses, gelartiges oder ein pulverförmiges Futtermittel (5) anliegen kann, wobei die Futterraumfläche (6) als eine erste Oberfläche der Barriere (3) in den Futterraum (4) weist, und in den Aufzuchtraum (8) weist die Aufzuchtfläche (10) als eine zweite Oberfläche der Barriere (3), wobei die Barriere (3) eine Vielzahl von Öffnungen (11) aufweist, deren Weite eine Passage der Insektenlarven (12) durch die Barriere (3) hindurch verhindern, jedoch den Zugang der Insektenlarven (12) zum Futtermittel (5) gewährleisten, **dadurch gekennzeichnet, dass** die Aufzuchtfläche (10) der Barriere (3) eine Fläche bildet, auf der die Insektenlarven (12) im Gebrauch leben.

2. Vorrichtung nach Anspruch 1, wobei die Barriere (3) als ein Lochblech, ein Gittergeflecht oder ein Abstandsgewirke ausgeführt ist.

3. Vorrichtung nach Anspruch 1 oder 2, wobei die Barriere (3) eine Neigung gegenüber der Horizontalen aufweist, die einen Winkel zwischen 5 und 30° aufweist und damit eine Trennung von Abscheidungen, Frass (14) und Häuten von den Insektenlarven (12) auf der Aufzuchtfläche (10) ermöglicht.

4. Vorrichtung nach einem der vorherigen Ansprüche, umfassend eine Vibrationseinrichtung (22), wobei die Barriere (3) so ausgeführt ist, dass sie zur Ernte der Insektenlarven (12) mittels einer der Vibrationseinrichtung (22) in Schwingungen versetzt wird.

5. Vorrichtung nach einem der vorherigen Ansprüche, wobei die Barriere (3) als ein Band bandförmig und damit kontinuierlich beweglich ausgeführt ist um Frass (14) und Häute abzuführen sowie Insektenlarven (12) zur Ernte auszuwerfen.

6. Vorrichtung nach Anspruch 1 oder 2, wobei die Barriere (3) einen unteren Abschluss einer weiterhin zumindest seitlich geschlossenen Insektenbox (7) bildet, wobei die Barriere im Gebrauch mit der Futterraumfläche (6) auf dem Futtermittel (5) aufsitzt und dieses durch die Öffnungen (11) hindurch von der Aufzuchtfläche (10) her durch die Insektenlarven (12) erreichbar ist.

7. Vorrichtung nach Anspruch 6, umfassend eine Vibrationseinrichtung (22), wobei die Vibrationseinrichtung (22) mit der Insektenbox (7) und/oder dem Aufzuchtbehälter (2) verbunden ist und ein Absinken der Insektenbox (7) im Futtermittel (5) durch Vibrationen unterstützt.

8. Vorrichtung nach einem der vorherigen Ansprüche, wobei die Barriere (3) eine Beschichtung aufweist oder aus einem Material besteht, das Schimmel und Abrieb verhindert, wobei das Material der Oberfläche der Barriere gebürsteter Edelstahl, feine Kartonage, Textil, glattes Polypropylen oder verzinktes Blech ist.

9. Vorrichtung nach einem der vorherigen Ansprüche, wobei eine Pumpe (18) zur Zufuhr des Futtermittels (5) aus einem Futtermitteltank (16) in den Futterraum (4) vorgesehen ist.

10. Verfahren zur Aufzucht von Insektenlarven, **dadurch gekennzeichnet, dass** ein Futtermittel (5) einer Vorrichtung (1) nach einem der Ansprüche 1 bis 9 kontinuierlich oder diskontinuierlich in einen Futterraum (4) zugeführt wird, sodass es durch Öffnungen (11) in einer Barriere (3) stets für die Insektenlarven (12) verfügbar ist, wobei kontinuierlich oder diskontinuierlich Frass (14) und Häute abgeführt sowie Insektenlarven (12) im Stadium vor der Verpuppung geerntet werden, wobei das Futtermittel (5) die für Insektenlarven (12) benötigten Nährstoffe und Wasser enthält, und wobei das Futtermittel (5) zu einem höherviskosen oder gelartigen Fluid, dessen Viskosität zwischen 104 MPa*s und 108 MPa*s beträgt, oder zu einem feuchten Pulver aufbereitet ist.

## Claims

1. Device (1) for rearing insect larvae, comprising a rearing container (2) and, arranged therein, a barrier (3), a feed space (4) and a rearing space (8), wherein the feed space (4) is separated from the rearing space (8) by the barrier (3), wherein the barrier (3) has a rearing surface (10) and a feed space surface (6) opposite the rearing surface (10), on which a feed (5) with a viscosity between 10⁴ MPa*s and 10⁸ MPa* higher viscosity, gel-like or powdered feed (5) can lie, wherein the feed space surface (6) faces into the feed space (4) as a first surface of the barrier (3), and the rearing space (8) faces the rearing space (8) as a second surface of the barrier (3), wherein the barrier (3) has a plurality of openings (11) whose width prevents the insect larvae (12) from passing through the barrier (3) but allows the insect larvae (12) to access the feed (5), **characterised in that** the rearing surface (10) of the barrier (3) forms a surface on which the insect larvae (12) live during use.

2. Device according to claim 1, wherein the barrier (3) is designed as a perforated sheet, a mesh or a spacer fabric.

3. Device according to claim 1 or 2, wherein the barrier (3) has an inclination relative to the horizontal which is between 5 and 30°, thereby enabling separation of excrement, frass (14) and skins from the insect larvae (12) on the rearing surface (10).

4. Device according to one of the previous claims, comprising a vibration device (22), wherein the barrier (3) is designed such that it is set into vibration by means of the vibration device (22) in order to harvest the insect larvae (12).

5. Device according to one of the previous claims, wherein the barrier (3) is designed as a continuous strip so as to be continuously movable in order to remove frass (14) and skins and to eject insect larvae (12) for harvesting.

6. Device according to claim 1 or 2, wherein the barrier (3) forms a lower closure of an insect box (7) which is further closed at least at the sides, wherein in use the barrier rests with the feed space surface (6) on the feed (5) and is accessible to the insect larvae (12) from the rearing surface (10) through the openings (11).

7. Device according to claim 6, comprising a vibration device (22), wherein the vibration device (22) is connected to the insect box (7) and/or the rearing container (2) and supports the sinking of the insect box (7) into the feed (5) by means of vibrations.

8. Device according to one of the previous claims, wherein the barrier (3) has a coating or is made of a material that prevents mould and abrasion, wherein the material of the surface of the barrier is brushed stainless steel, fine cardboard, textile, smooth polypropylene or galvanised sheet metal.

9. Device according to one of the previous claims, wherein a pump (18) is provided for supplying the feed (5) from a feed tank (16) into the feed space (4).

10. Method for rearing insect larvae, **characterised in that** a feed (5) of a device (1) according to one of claims 1 to 9 is continuously or discontinuously fed into a feed space (4) so that it is always available to the insect larvae (12) through openings (11) in a barrier (3), wherein frass (14) and skins are continuously or discontinuously removed and insect larvae (12) are harvested in the stage prior to pupation, wherein the feed (5) contains the nutrients and water required for insect larvae (12), and wherein the feed (5) is processed into a higher-viscosity or gel-like fluid whose viscosity is between 104 MPa*s and 108 MPa*s, or into a moist powder.

## Revendications

1. Dispositif (1) pour l'élevage de larves d'insectes, comprenant un récipient d'élevage (2) et une barrière (3) disposée en son sein, un espace de nourriture (4) et un espace d'élevage (8), dans lequel l'espace de nourriture (4) est séparé de l'espace d'élevage (8) par la barrière (3), dans lequel la barrière (3) présente une surface d'élevage (10) et une surface d'espace de nourriture (6) opposée à la surface d'élevage (10), sur laquelle un aliment pour animaux (5) à viscosité supérieure, de type gel ou pulvérulent avec une viscosité comprise entre 10⁴ MPa*s et 10⁸ MPa*s peut reposer, dans lequel la surface d'espace de nourriture (6) est dirigée dans l'espace de nourriture (4) en tant que première surface de la barrière (3), et la surface d'élevage (10) est dirigée dans l'espace d'élevage (8) en tant que seconde surface de la barrière (3), dans lequel la barrière (3) présente une pluralité d'ouvertures (11) dont la largeur empêche un passage des larves d'insectes (12) à travers la barrière (3), mais garantissent l'accès des larves d'insectes (12) à l'aliment pour animaux (5), **caractérisé en ce que** la surface d'élevage (10) de la barrière (3) forme une surface sur laquelle les larves d'insectes (12) vivent en cours d'usage.

2. Dispositif selon la revendication 1, dans lequel la barrière (3) est réalisée en tant que tôle perforée, treillis grillagé ou maille espacée.

3. Dispositif selon la revendication 1 ou 2, dans lequel la barrière (3) présente une inclinaison par rapport à l'horizontale qui présente un angle compris entre 5 et 30° et permet ainsi une séparation de dépôts, nourriture (14) et peaux des larves d'insectes (12) sur la surface d'élevage (10).

4. Dispositif selon une des revendications précédentes, comprenant une installation de vibration (22), dans lequel la barrière (3) est réalisée de sorte qu'elle est amenée en oscillations au moyen de l'installation de vibration (22) pour la récolte des larves d'insectes (12).

5. Dispositif selon une des revendications précédentes, dans lequel la barrière (3) est réalisée en forme de bande en tant que bande et ainsi de manière mobile en continu pour évacuer de la nourriture (14) et des peaux ainsi qu'éjecter des larves d'insectes (12) pour la récolte.

6. Dispositif selon la revendication 1 ou 2, dans lequel la barrière (3) forme une fermeture inférieure d'une boîte à insectes (7) fermée en outre au moins latéralement, dans lequel la barrière repose en cours d'usage avec la surface d'espace de nourriture (6) sur l'aliment pour animaux (5) et celui-ci peut être atteint par les larves d'insectes (12) à travers les ouvertures (11) depuis la surface d'élevage (10).

7. Dispositif selon la revendication 6, comprenant une installation de vibration (22), dans lequel l'installation de vibration (22) est connectée à la boîte à insectes (7) et/ou au récipient d'élevage (2) et favorise un abaissement de la boîte à insectes (7) dans l'aliment pour animaux (5) par des vibrations.

8. Dispositif selon une des revendications précédentes, dans lequel la barrière (3) présente un revêtement ou se compose d'un matériau qui empêche la moisissure et l'abrasion, dans lequel le matériau de la surface de la barrière est de l'acier inoxydable brossé, du cartonnage fin, du textile, du polypropylène lisse ou de la tôle zinguée.

9. Dispositif selon une des revendications précédentes, dans lequel une pompe (18) est prévue pour l'amenée de l'aliment pour animaux (5) d'un réservoir d'aliment pour animaux (16) dans l'espace de nourriture (4).

10. Procédé d'élevage de larves d'insectes, **caractérisé en ce qu'**un aliment pour animaux (5) est amené à un dispositif (1) selon une des revendications 1 à 9 en continu ou de manière discontinue dans un espace de nourriture (4) de sorte qu'il est toujours disponible pour les larves d'insectes (12) à travers des ouvertures (11) dans une barrière (3), dans lequel de la nourriture (14) et des peaux sont évacuées en continu ou de manière discontinue ainsi que des larves d'insectes (12) au stade de la nymphose sont récoltées, dans lequel l'aliment pour animaux (5) contient les nutriments nécessaires pour des larves d'insectes (12) et de l'eau, et dans lequel l'aliment pour animaux (5) est préparé en un fluide à viscosité supériere ou de type gel dont la viscosité est comprise entre 10⁴ MPa*s et 10⁸ MPa*s, ou en une poudre humide.
